(19) Europäisches Patentamt
European Patent Office
Office européen des brevets

(11) **EP 4 717 165 A1**

(12) **DEMANDE DE BREVET EUROPEEN**

(43) Date de publication:
**01.04.2026 Bulletin 2026/14**

(21) Numéro de dépôt: **25204789.9**

(22) Date de dépôt: **25.09.2025**

(51) Classification Internationale des Brevets (IPC):
**A61B 5/12** *(2006.01)* **H04R 29/00** *(2006.01)*

(52) Classification Coopérative des Brevets (CPC):
**A61B 5/123;** E04B 1/84; H04R 3/005; H04R 5/027

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **25.09.2024 FR 2410263**

(71) Demandeur: **A2S**
**69960 Corbas (FR)**

(72) Inventeurs:
• **BENARROUS, Edmond**
**69960 Corbas (FR)**
• **FUMEY, Franck**
**69960 Corbas (FR)**

(74) Mandataire: **Innovincia**
**11, avenue des Tilleuls**
**74200 Thonon-Les-Bains (FR)**

(54) **PROCÉDÉ D'ÉVALUATION AUDITIVE D'UN ESPACE PRÉDÉFINI**

(57) L'invention concerne un procédé d'évaluation auditive d'un espace prédéfini (2) comprenant les étapes suivantes :
- prendre avec un capteur acoustique (1), dans l'espace prédéfini (2), un échantillon acoustique sur une durée prédéfinie,
-convertir l'échantillon en un flux de données correspondant à un flux de signaux arrivant au cerveau avec des canaux fréquentiels, - évaluer pour chaque canal le flux de signaux selon une échelle de valeur biologique en déterminant un indice de nuisance acoustique pour chaque canal,
- sélectionner un profil de caractérisation de ladite personne comprenant des caractéristiques physiologiques, temporelles et psychiques,
- sélectionner un profil de caractérisation de l'espace comprenant des caractéristiques d'une activité présente dans l'espace et d'un lieu,
- fournitures des indices de nuisance acoustique des canaux ainsi que du profil de caractérisation de ladite personne et du profil de caractérisation de l'espace en entrée d'un réseau neuronal artificiel.

[Fig 2]

Figure 2

**Description**

L'invention concerne le domaine de l'audition et plus spécifiquement un procédé d'évaluation auditive d'un espace prédéfini par simulation de la perception acoustique d'une personne dans cet espace en vue de déterminer l'existence d'une gêne acoustique pour cette personne dans cet espace.

Une partie de plus en plus importante de la population mondiale est exposée à des niveaux sonores nocifs pour l'ouïe à cause de l'augmentation du nombre et du niveau des émissions acoustiques environnementales. Devant la multiplication des pathologies liées au bruit, de plus en plus de secteurs d'activités et de loisirs prennent petit à petit conscience de l'impact des nuisances sonores sur la santé. Que ce soit dans la sphère professionnelle ou privée, s'en soucier peut permettre aussi d'améliorer grandement la qualité de vie de chacun, et ce particulièrement au travail.

Le bruit est d'ailleurs reconnu en France comme cause de maladie professionnelle depuis 1963 et les surdités professionnelles se situent au quatrième rang des maladies professionnelles (source CIDB). Par ailleurs on doit avoir à l'esprit que le coût social du bruit est loin d'être négligeable. En France il a été évalué à 57 milliards d'euros, toutes origines confondues (transports, voisinage, milieu professionnel, milieu scolaire). Selon une enquête IFOP/JNA, le bruit et les nuisances sonores seraient à l'origine d'une perte de productivité d'environ 23 milliards d'euros par an. Ces quelques données sanitaires et économiques montrent que la pollution sonore est un enjeu crucial pour l'évolution de notre société.

Le système auditif humain et soumis à des stimuli permanents, c'est le seul sens qui ne se repose pas. Le cerveau humain va ainsi recevoir continuellement des informations liées à toutes les sources acoustiques présentes dans notre "sphère" sonore, et restituer des sensations qui sont de l'ordre du quantitatif mais aussi du subjectif, et à cet égard qui peuvent sortir du cadre d'une démarche purement rationnelle. La sensation auditive s'accompagne donc automatiquement d'une évaluation psychoacoustique qui intègre principalement deux types de facteurs :

- Des facteurs auditifs qui sont liés au système auditif humain et dépendent de la façon dont l'ouïe et le cerveau de l'individu perçoivent 'physiquement' les stimuli sonores.
- Des facteurs émotionnels ressentis en parallèle et qui prennent en compte l'expérience psychologique, sociale et culturelle de l'individu, son état émotionnel et son activité momentanée.

D'un point de vue neuroscientifique la sensation auditive peut être définie en toute généralité comme la transformation d'un évènement sonore extérieur en activité neuronale interne au cerveau. C'est la première étape d'une chaîne d'événements physiques, biochimiques et neurologiques allant de la stimulation énergétique d'un organe sensoriel, en l'occurrence l'oreille, à la réception inconsciente qu'en fait le cerveau, alors que la perception sonore correspond à un ensemble de processus neuronaux par lesquels l'humain en prend conscience. La sensation auditive correspond donc à l'information brute décryptée par le cerveau et à cet égard peut être assimilée à une émotion tandis que la perception résultant d'un processus cognitif complexe supplémentaire peut être assimilée à un sentiment d'émotion, une "prise de conscience"

La gêne acoustique ou nuisance sonore est une information auditive parasite procurant une interférence neuronale néfaste à la réalisation d'une activité cérébrale principale, et perçue émotivement comme déplaisante.

Toutefois, la difficulté d'évaluation d'une gêne acoustique réside par exemple dans le fait que l'écoute de la musique en concert à un fort niveau n'est pas mal perçue par les spectateurs, ni le coup d'un fusil pour un chasseur participant à par exemple à un jeu de balltrap, bien que dans ces cas on sache pertinemment que l'oreille subit des agressions importantes. Par ailleurs force est de constater que la gêne n'est pas toujours associée à un niveau sonore élevé. On note par exemple comme très déplaisant et gênant le bruit du goutte à goutte d'un robinet qui fuit, le bruit lancinant d'un climatiseur, la discussion d'un voisin de bureau....

Dans certains cas l'émergence tonale prononcée et continue (bruit concentré sur certaines fréquences) peut déclencher un phénomène de prégnance dans l'activité du cerveau en saturant certaines voies neuronales et conduire à une situation de gêne obsessionnelle (bruit d'aspirateur, de voisinage...) sans pour cela être d'un niveau très élevé.

Les réglementations acoustiques actuellement en vigueur ne permettant pas appréhender cette gêne acoustique.

La présente invention vise à évaluer le phénomène de gêne acoustique, notamment par une approche plus fiable et objective que le permettent les méthodes connues dans l'état de la technique et qui se basent uniquement sur des caractéristiques physiques du signal sonore comme la pression acoustique et/ou la fréquence.

A cet effet, l'invention concerne un procédé d'évaluation auditive d'un espace prédéfini par simulation de la perception acoustique d'une personne dans cet espace en vue de déterminer l'existence d'une gêne acoustique pour cette personne, comprenant les étapes suivantes :

- prendre avec au moins un capteur acoustique, notamment un capteur acoustique binaural, dans l'espace prédéfini un échantillon acoustique sur une durée prédéfinie d'au moins une minute,
- convertir l'échantillon acoustique en un flux de données correspondant à un flux de signaux du type neuronal arrivant au cerveau avec des canaux fréquentiels compris entre 16Hz et 16000Hz, le flux de signaux du type neuronal pour chaque canal fréquentiel étant formé par des impulsions dont la fréquence de répétition par seconde est une fonction de l'intensité du signal acoustique du canal fréquentiel considéré

- évaluer pour chaque canal le flux de signaux du type neuronal selon une échelle de valeur biologique en déterminant un indice de nuisance acoustique pour chaque canal,

- sélectionner un profil de caractérisation de ladite personne comprenant des caractéristiques physiologiques, temporelles et psychiques,

- sélectionner un profil de caractérisation de l'espace comprenant des caractéristiques d'une activité présente dans l'espace et d'un lieu,

- fournitures des indices de nuisance acoustique des canaux fréquentiels ainsi que du profil de caractérisation de ladite personne et du profil de caractérisation de l'espace en entrée d'un réseau neuronal artificiel pour évaluer de manière auditive l'espace prédéfini par une classification selon au moins une première catégorie correspondant à la survenue d'une gêne acoustique perçue par la personne et une seconde catégorie correspondant à la perception d'une absence d'une gêne acoustique perçue par la personne.

Le procédé peut présenter un ou plusieurs des aspects suivants pris seuls ou en combinaison :
On utilise par exemple 620 canaux fréquentiels compris entre 16Hz et 16000 Hz.

La fréquence de répétition est notamment comprise entre 0 et 330 par seconde.

Les caractéristiques physiologiques comprennent notamment au moins un des paramètres suivants : l'âge, notamment une tranche d'âge, le sexe.

Les caractéristiques temporelles comprennent par exemple au moins un des paramètres suivants : mois de l'année, tranche horaire de la journée telle que matin, après-midi, soirée ou nuit.

Les caractéristiques psychiques peuvent comprendre au moins un des paramètres suivants : un état attentionnel tel que éveillé, somnolent ou endormi et un état émotionnel tel que calme/ serein, stressé /angoissé, joyeux / gai, triste / mélancolique ou irrité / en colère.

L'échelle de valeur biologique peut être représentée par une fonction du type sigmoïde :

$$f(x) = \frac{1}{1 + e^{(-\alpha(x - x_0)}},$$

où x est l'intensité d'un stimulus, f(x) la réponse physiologique, $\alpha$ un facteur de sensibilité contrôlant la loi d'évolution et $x_0$ le point de basculement.

Le facteur de sensibilité $\alpha$ est notamment déterminé empiriquement par rapport à un protocole de test.

Les caractéristiques de l'activité comprennent par exemple au moins un des paramètres suivants : activité manuelle, sportive, ludique, musicale, informatique, lecture, écriture, discussion, méditation / réflexion.

Les caractéristiques de lieu comprennent en particulier au moins un des paramètres suivants : bureau, école, salle de réunion, atelier, moyen de transport, salle de spectacle, salle de restaurant, salle de sport, plein air.

L'invention concerne également un produit de programme informatique comprenant une ou plusieurs séquences d'instructions stockées accessibles à un processeur et qui, lorsqu'il est exécuté par le processeur, permet à ce dernier d'effectuer les étapes du procédé tel que décrit ci-dessus.

L'invention concerne aussi un procédé de réduction d'une gêne acoustique perçue par une personne dans un espace prédéfini, dans lequel

- selon une première étape, on procède à l'évaluation auditive de l'espace prédéfini selon un procédé tel que décrit ci-dessus,
- si le résultat de la première étape est classé selon une catégorie correspondant à la survenue d'une gêne acoustique perçue par la personne, on procède à la modification dudit espace, notamment par la pose / disposition d'éléments d'absorption acoustique dans cet espace,
- on réitère les première et seconde étapes jusqu'à obtenir comme résultat de la première étape un classement selon une catégorie correspondant à l'absence d'une gêne acoustique.

L'invention concerne de plus un dispositif de mise en œuvre d'un procédé tel que décrit ci-dessus, comprenant :

- au moins un capteur acoustique, notamment un capteur acoustique binaural, configuré pour pouvoir prendre dans un espace prédéfini un échantillon acoustique sur une durée prédéfinie d'au moins une minute,
- un convertisseur de l'échantillon acoustique en un flux de données correspondant à un flux de signaux du type neuronal arrivant au cerveau avec des canaux fréquentiels compris entre 16Hz et 16000Hz, le flux de signaux du type neuronal pour chaque canal fréquentiel étant formé par des impulsions dont la fréquence de répétition par seconde est une fonction de l'intensité du signal acoustique du canal fréquentiel considéré
- une unité d'évaluation pour évaluer pour chaque canal le flux de signaux du type neuronal selon une échelle de valeur biologique en déterminant un indice de nuisance acoustique pour chaque canal,

- une première unité de sélection configurée pour sélectionner un profil de caractérisation de ladite personne comprenant des caractéristiques physiologiques, temporelles et psychiques,

- une seconde unité de sélection configurée pour sélectionner un profil de caractérisation de l'espace comprenant des caractéristiques d'une activité présente dans l'espace et d'un lieu,

- une unité de traitement à réseau neuronal artificiel configurée pour recevoir en entrée des indices de nuisance acoustique des canaux fréquentiels ainsi que des profils de caractérisation de ladite personne et de caractérisation de l'espace en entrée, l'unité de traitement à réseau neuronal artificiel étant configurée pour évaluer de manière auditive l'espace prédéfini par une classification selon au moins une première catégorie correspondant à la survenue d'une gêne acoustique perçue par la personne et une seconde catégorie correspondant à la perception d'une absence d'une gêne acoustique perçue par la personne.

Le dispositif peut présenter une ou plusieurs des aspects suivants pris seuls ou en combinaison :

Les canaux fréquentiels compris entre 16Hz et 16000 Hz comprennent par exemple 620 canaux.

La fréquence de répétition du convertisseur de l'échantillon acoustique est notamment comprise entre 0 et 330 par seconde pour chaque canal.

Les caractéristiques physiologiques comprennent en particulier au moins un des paramètres suivants : l'âge, notamment une tranche d'âge, le sexe.

Les caractéristiques temporelles comprennent par exemple au moins un des paramètres suivants : mois de l'année, tranche horaire de la journée telle que matin, après-midi, soirée ou nuit.

Les caractéristiques psychiques comprennent notamment au moins un des paramètres suivants : un état attentionnel tel que éveillé, somnolent ou endormi et un état émotionnel tel que calme/ serein, stressé /angoissé, joyeux / gai, triste / mélancolique ou irrité / en colère.

L'échelle de valeur biologique de l'unité d'évaluation biologique peut être représentée par une fonction du type sigmoïde :

$$f(x) = \frac{1}{1+e^{(-\propto(x-x_0)}},$$

où x est l'intensité d'un stimulus, f(x) la réponse physiologique, $\alpha$ un facteur de sensibilité contrôlant la loi d'évolution et $x_o$ le point de basculement.

D'autres caractéristiques et avantages de l'invention ressortiront clairement de la description qui en est faite ci-après, à titre indicatif et nullement limitatif, en référence aux dessins annexés, dans lesquels :

[Fig 1] La figure 1 est un organigramme montrant différentes étapes du procédé selon la présente invention selon un premier mode réalisation, et
[Fig.2] La figure 2 est un schéma simplifié d'un mode de réalisation d'un dispositif pour la mise en œuvre d'un procédé selon la présente invention selon un premier mode réalisation.
[Fig.3] La figure 3 est un organigramme montrant différentes étapes d'un procédé de réduction d'une gêne acoustique perçue par une personne dans un espace prédéfini.

Sur les différentes figures, les éléments identiques portent les mêmes numéros de référence. Les réalisations suivantes sont des exemples.

Dans la présente description, on peut indexer certains éléments ou paramètres, comme par exemple premier élément ou deuxième élément ainsi que premier paramètre et second paramètre ou encore premier critère et deuxième critère, etc. Dans ce cas, il s'agit d'un simple indexage pour différencier et dénommer des éléments ou paramètres ou critères proches, mais non identiques. Cette indexation n'implique pas une priorité d'un élément, paramètre ou critère par rapport à un autre et on peut aisément interchanger de telles dénominations sans sortir du cadre de la présente description.

Le procédé de l'invention peut être mise en œuvre au moins en partie sur un ordinateur. Dans ce contexte, sauf indication contraire, il est entendu que, tout au long de la présente description, les discussions utilisant des termes tels que « informatique », « calcul » et « génération » ou similaires, se réfèrent à l'action et/ou aux processus d'un ordinateur ou d'un système informatique, ou d'un dispositif informatique électronique similaire, qui manipule et/ou transforme des données représentées sous forme de quantités physiques, telles que des quantités électroniques, dans les registres et/ou les mémoires du système informatique en d'autres données représentées de manière similaire sous forme de quantités physiques dans les mémoires, les registres ou d'autres dispositifs de stockage, de transmission ou d'affichage de l'information du système informatique.

Un produit de programme informatique comprenant une ou plusieurs séquences d'instructions stockées accessibles à un processeur et qui, lorsqu'il est exécuté par le processeur, permet à ce dernier d'effectuer les étapes du procédé est également proposé.

Un tel programme informatique peut être stocké sur un support de stockage lisible par ordinateur, tel que, mais sans s'y limiter, tout type de disque, y compris les disquettes, les disques optiques, les CD-ROM, les disques magnétiques-optiques, les mémoires mortes (ROM), les mémoires vives (RAM), les mémoires mortes programmables électriquement (EPROM), les mémoires mortes effaçables et programmables électriquement (EEPROM), les cartes magnétiques ou optiques, ou tout autre type de support adapté au stockage d'instructions électroniques, et capable d'être couplé à un bus de

Un support lisible par ordinateur contenant une ou plusieurs séquences d'instructions du produit de programme informatique est donc proposé. Cela permet de mettre en œuvre la méthode en tout lieu.

Les procédés et les affichages présentés ici ne sont pas intrinsèquement liés à un ordinateur particulier ou à un autre appareil. Divers systèmes d'usage général peuvent être utilisés avec des programmes conformes aux enseignements du présent document, ou il peut s'avérer commode de construire un appareil plus spécialisé pour exécuter la méthode souhaitée. La structure souhaitée pour une variété de ces systèmes apparaîtra dans la description ci-dessous. En outre, les modes de réalisation de la présente invention ne sont pas décrits en référence à un langage de programmation particulier. Il sera apprécié qu'une variété de langages de programmation puisse être utilisée pour mettre en œuvre les enseignements des inventions telles que décrites ici.

On va maintenant décrire le procédé d'évaluation auditive d'un espace prédéfini 2 par simulation de la perception acoustique d'une personne dans cet espace en vue de déterminer l'existence d'une gêne acoustique pour cette personne et le dispositif associé,

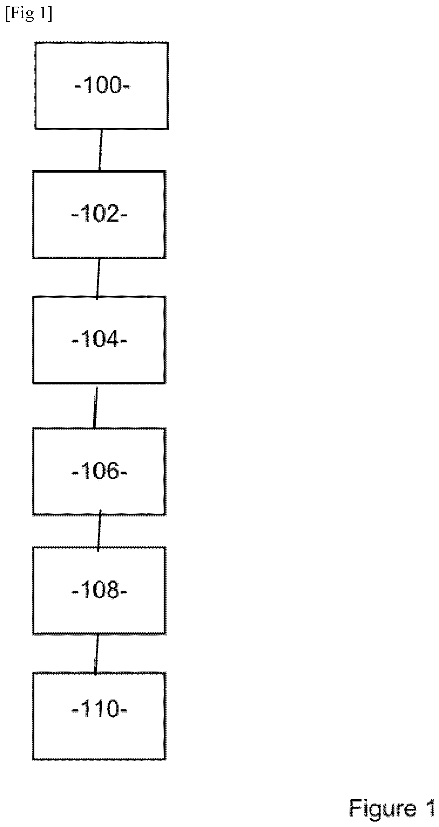

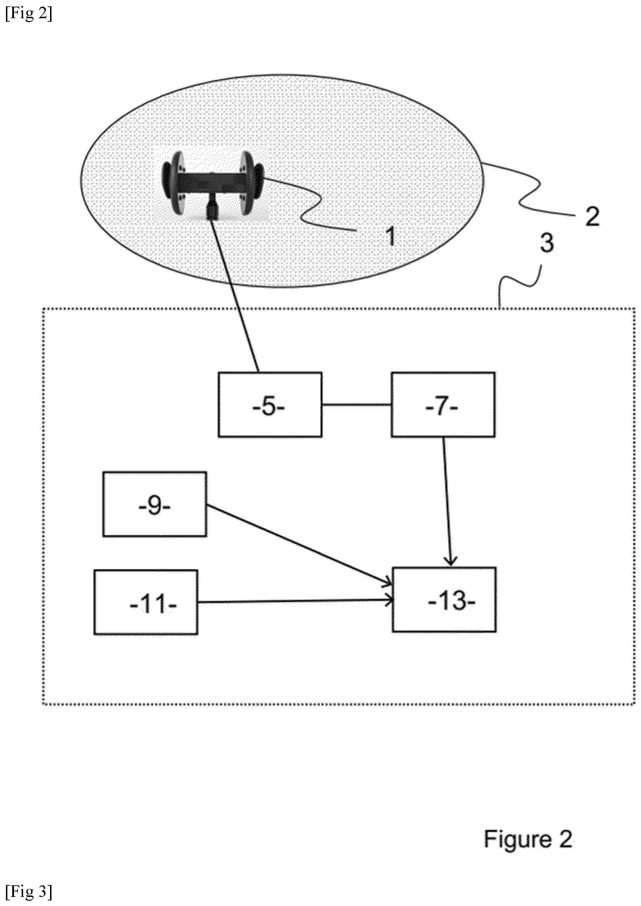

Un possible de mode de réalisation de la présente invention sera décrit au regard des figures 1 et 2 dont la figure 1 montre un organigramme montrant différentes étapes du procédé selon la présente invention selon un premier mode réalisation et la figure 2 présente un schéma simplifié d'un mode de réalisation d'un dispositif pour la mise en œuvre d'un procédé.

Comme on le voit sur la figure 2, le dispositif de mise en œuvre du procédé selon l'invention comprend au moins un capteur acoustique 1. Ce capteur 1 est configuré pour pouvoir prendre selon une première étape 100 (voir figure 1) dans un espace prédéfini 2 un échantillon acoustique sur une durée prédéfinie d'au moins une minute.

Un capteur acoustique 1 peut comprendre un seul microphone. Selon l'exemple décrit, il s'agit par exemple d'un capteur acoustique binaural comprenant par exemple deux microphones espacés notamment d'une distance qui correspond à la distance moyenne entre deux oreilles d'un humain et dont les parties sensibles sont dirigés dans des directions opposées.

Le capteur binaural 1 permet un enregistrement binaural qui est basé sur une réplique du système auditif mettant en œuvre un ensemble de microphones disposés de manière à pouvoir recréer avec précision la différence de temps et d'intensité perçue entre les deux oreilles d'un humain.

L'espace prédéfini 2 est par exemple un espace dont la qualité acoustique doit être évaluée. Il peut s'agir par exemple d'une pièce dans un bâtiment, un bureau, une salle, un lieu de travail fermé dans un espace de production d'une usine, mais aussi d'un endroit un plein air.

L'échantillon acoustique est un ou plusieurs enregistrement(s) sur une durée prédéfinie d'au moins une minute. Il peut aussi s'agir de plusieurs échantillons prises à des moments différents et agrégés, par exemple le matin, le midi et le soir ou des échantillons prises à des endroits différents de la pièce.

Dans le cas d'un capteur acoustique binaural 1, on dispose par exemple de deux échantillons acoustiques qui peuvent être traités en parallèle de façon indépendante.

Le capteur acoustique binaural 1 est par exemple relié de façon filaire ou non filaire à un ordinateur 3 spécifique qui comprend par exemple une entrée pour recevoir et traiter les signaux issus du capteur binaural 1.

Ainsi, l'ordinateur 3 comprend par exemple un convertisseur 5 configuré pour convertir lors d'une étape 102 le ou les échantillon(s) acoustique(s) en un flux de données correspondant à un flux de signaux du type neuronal arrivant au cerveau avec des canaux fréquentiels compris entre 16Hz et 16000Hz. Le flux de signaux du type neuronal pour chaque canal fréquentiel est formé par des impulsions dont la fréquence de répétition par seconde est une fonction de l'intensité du signal acoustique du canal fréquentiel considéré.

Il s'est avéré que l'être humain est capable de discerner environ 620 canaux de fréquence (140 en dessous de 500 Hz et 420 en dessus de 500 Hz).

On peut considérer que chaque canal correspond à un canal neuronal du cerveau humain. En conséquence, l'échantillon acoustique est traité de façon à couper la plage de fréquence entre 16Hz et 16000Hz en 620 canaux fréquentiels.

Bien entendu, on peut diminuer ou augmenter le nombre de canaux fréquentiels pour la présente méthode sans sortir du cadre de la présente invention.

Selon la présente méthode, le traitement et en particulier la conversion en un flux de signaux du type neuronal des divers canaux est réalisée individuellement pour chaque canal fréquentiel. Les canaux fréquentiels sont notamment traités en parallèle.

Pour ce faire l'échantillon acoustique pour chaque canal fréquentiel est converti en une valeur entre 0 et 330 ce qui correspond à une réponse biologique comprise entre 0 et 330 potentiels d'actions (ou « PA » ).

En effet, un élément fondamental de la communication neuronale est le Potentiel d'Action (PA). Un neurone qui vient d'être stimulé (allumé) transmet un message à un autre neurone en envoyant à travers son axone des impulsions électrochimiques appelées "potentiel d'action" (PA).

L'amplitude des impulsions émises par un neurone est à peu près constante et donc l'information véhiculée se trouver dans leur fréquence d'apparition ou de répétition. Eu égard à leur durée, les impulsions nerveuses ne peuvent se suivre à moins de 3 millisecondes d'intervalle les unes des autres. A cet égard cette durée peut être considérée comme l'unité naturelle de temps de traitement de l'information nerveuse dans le cerveau. On peut en déduire que la fréquence limite maximale de décharge d'un neurone, et donc de la communication neuronale, est d'environ 300-400 Hz par voie neuronale, d'où une échelle de valeur biologique comprise entre 0 et 330, 330 correspondant alors au nombre maximal d'impulsions pouvant être émis par un neurone. La fréquence de répétition par seconde ou potentiel d'action est donc une fonction de l'intensité du signal acoustique du canal fréquentiel considéré.

Compte tenu des éléments exposés précédemment, l'information neuronale de l'ouïe peut donc être exprimée par une matrice 620 x 330, correspondant à 620 voies parallèles échantillonnant le domaine fréquentiel audible (entre 16Hz et 16000 Hz), et 330 niveaux échantillonnant la valeur biologique de la force sonore (entre 0 et 330 Potentiels d'Action par seconde).

Pour chaque canal neuronale identifié par une position sur la membrane basilaire et associée à une fréquence sonore, le nombre de potentiels d'action va ainsi osciller entre 0 et une valeur maximum qui a été située à 330 PA/s compte tenu de la durée d'un PA typique, et donner lieu à une perception sonore dont l'évolution répond à une sensation telle que décrite plus haut, dans les limites physiques délimitées par le seuil d'audition et le seuil de douleur, qui sont des situations extrêmes qui bornent la réponse sensorielle de l'ouïe.

Puis selon une étape 104, on évalue à l'aide d'une unité d'évaluation 7 (voir figure 2) pour chaque canal le flux de signaux du type neuronal selon une échelle de valeur biologique en déterminant un indice de nuisance acoustique pour chaque canal.

L'échelle de valeur biologique est par exemple représentée par une fonction du type sigmoïde :

$$f(x) = \frac{1}{1+e^{(-\alpha(x-x_0)}}$$

où x est l'intensité d'un stimulus, f(x) la réponse physiologique, $\alpha$ un facteur de sensibilité contrôlant la loi d'évolution et $x_o$ le point de basculement.

Généralement pour un canal, le un facteur $\alpha$ de sensibilité est compris entre 0,08 et 0,15 et $x_o$ le point de basculement est exprimé de façon nondimensionnelle vaut donc 0,5. Le facteur de sensibilité $\alpha$ peut aussi être déterminé empiriquement par rapport à un protocole de test.

Le facteur $\alpha$ de la fonction sigmoïde peut être assimilé à sensibilité neuronale globale d'un individu. Le point de basculement $x_o$ de la fonction sigmoïde est assimilé à une valeur médiane de la dynamique spectrale de l'oreille.

L'intensité x du stimulus est donc une valeur entre 0 et 330 pour chaque canal.

Il s'est montré que les valeurs biologiques peuvent être représentés par une loi d'évolution universelle de type « sigmoïde » en fonction de l'amplitude de la stimulation. Une telle loi traduit que par exemple lorsqu'on est soumis à un bruit de faible niveau sonore, on n'est pas très conscient de la gêne occasionnée et que passé un certain seuil la gêne est avérée puis empire rapidement pour atteindre un niveau insupportable en suivant approximativement cette évolution.

Cela permet aussi de traduire que le début une sensation provenant un stimulus acoustique peut paraître confuse car noyée dans un "bruit de fond neuronal", mais qui peut devenir de plus en plus émergente puis culmine pour aboutir à une zone de saturation pour laquelle la sensation ne varie que faiblement sans que l'on sache précisément où se trouve la limite à mesure que le stimulus augmente en intensité. C'est par exemple ce qu'on observe dans le domaine de l'acoustique pour la perception de la force sonore où dans un tout autre domaine pour la perception de la douleur. Le point de basculement de cette fonction (là où la dérivée atteint son maximum) peut être vu comme un seuil statistique au-delà duquel la sensation est avérée et où en a pris pleine conscience.

Puis l'ordinateur 3 peut comprendre une première unité de sélection 9 configurée pour sélectionner selon une étape 106 un profil de caractérisation de ladite personne comprenant des caractéristiques physiologiques, temporelles et psychiques ainsi qu'une seconde unité de sélection 11 configurée pour sélectionner selon une étape 108 un profil de caractérisation de l'espace comprenant des caractéristiques d'une activité présente dans l'espace et d'un lieu.

Les caractéristiques physiologiques comprennent au moins un des paramètres suivants : l'âge, notamment une tranche d'âge, le sexe.

Les caractéristiques temporelles comprennent au moins un des paramètres suivants : mois de l'année, tranche horaire de la journée telle que matin, après-midi, soirée ou nuit.

Les caractéristiques psychiques comprennent au moins un des paramètres suivants : un état attentionnel tel que éveillé, somnolent ou endormi et un état émotionnel tel que calme/ serein, stressé /angoissé, joyeux / gai, triste / mélancolique ou irrité / en colère.

Les caractéristiques de l'activité comprennent au moins un des paramètres suivants : activité manuelle, sportive, ludique, musicale, informatique, lecture, écriture, discussion, méditation / réflexion.

Les caractéristiques de lieu comprennent au moins un des paramètres suivants : bureau, école, salle de réunion, atelier, moyen de transport, salle de spectacle, salle de restaurant, salle de sport, plein air.

Toutes ces caractéristiques peuvent par exemple être saisies via une interface d'ordinateurs avec par exemple un écran et une souris permettant de sélectionner ces caractéristiques dans des menus déroulants.

L'ordinateur comprend en outre une unité 13 de traitement à réseau neuronal artificiel configurée pour recevoir en entrée des indices de nuisance acoustique pour chaque canal ainsi que des profils de caractérisation de ladite personne et de caractérisation de l'espace en entrée.

Bien entendu, les unités 5, 7, 9, 11 et 13 peuvent faire partie d'un ou de plusieurs ordinateurs. Il peut s'agir d'unités physiques distinctes ou non. Plus spécifiquement, il peut s'agir de routines de software pour traiter ou réaliser une ou plusieurs étapes telles que décrites ci-dessus.

L'unité 13 de traitement est configurée pour évaluer lors d'une étape 110 de manière auditive l'espace prédéfini 2 par une classification selon au moins une première catégorie correspondant à la survenue d'une gêne acoustique perçue par la personne et une seconde catégorie correspondant à la perception d'une absence d'une gêne acoustique perçue par la personne.

L'évaluation finale de la gêne associée à un stimulus sonore a été réalisée par l'unité de traitement 13 en déterminant un indice de nuisance acoustique compris entre 0 et 100%. Pour ce faire l'unité 13 de traitement à réseau neuronal artificiel prend en compte en particulier les canaux fréquentiels ayant le nombre d'impulsions par seconde ou de PA/s le plus élevé.

On peut distinguer cinq plages de valeurs de l'indice de nuisance acoustique correspondant à des intervalles : [0,00-0,25[ ; [0,25-0,75[ ; [0,75-0,95[ ; [0,95-1,00] à l'échelle verbale de nuisance acoustique suivante : [très faible; faible; moyenne; forte; très forte].

Les plages très faible; faible; moyenne sont par exemple assimilées à la seconde catégorie correspondant à la perception d'une absence d'une gêne acoustique perçue par la personne et les plages forte; très forte à la première catégorie correspondant à la survenue d'une gêne acoustique perçue par la personne.

Ainsi, on peut évaluer une gêne acoustique de manière plus fiable et avec plus de précision.

Le procédé et dispositif décrit ci-dessus peuvent être mis à profit pour réduire une gêne acoustique perçue par une personne dans un espace prédéfini 2.

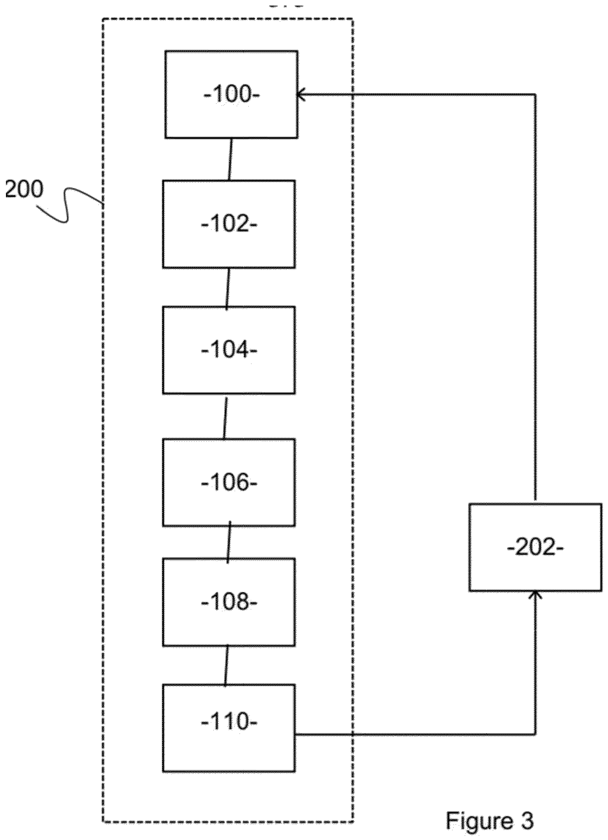

En référence à la figure 3, pour réaliser une réduction d'une gêne acoustique perçue par une personne dans un espace prédéfini 2, selon une première étape 200, on procède à l'évaluation auditive de l'espace prédéfini 2 comme décrit ci-dessus avec les étapes 100 à 110 (Les étapes 100 à 110 sont identiques à celles des étapes 100 à 110 de la figure 1).

Puis, si le résultat de la première étape 200 classe le résultat d'évaluation selon une catégorie correspondant à la survenue d'une gêne acoustique perçue par la personne, on procède selon une étape 202 lors de laquelle on procède à la modification dudit espace.

Une telle modification consiste par exemple par la pose / ou disposition d'éléments d'absorption acoustique dans cet espace, comme par exemple des panneaux d'absorption acoustique. Pour ce faire, on tient en particulier compte par exemple de la position du capteur binaural 1 dans l'espace prédéfini 2. Ainsi, on peut par exemple disposer des panneaux d'absorption acoustique autour de la position du capteur binaural 1 dans l'espace prédéfini 2 pour créer un espace protégé par une isolation / atténuation acoustique autour de cet espace ou en disposant des panneaux d'isolation acoustique ou d'atténuation acoustique sur des murs ou au niveau du plafond.

Puis, on réitère les première et seconde étapes 200 et 202 jusqu'à obtenir comme résultat de la première étape 200 un classement selon une catégorie correspondant à l'absence d'une gêne acoustique. Un tel procédé est intéressant car il est possible d'optimiser le nombre de panneaux d'isolation acoustique à disposer.

On peut ainsi améliorer la qualité acoustique d'un espace prédéfini 2 de manière plus objective en tenant compte plus précisément du processus neuronal de formation du son dans le cerveau humain et des réactions potentiels du cerveau à une stimulation acoustique.

**Revendications**

1. Procédé d'évaluation auditive d'un espace prédéfini (2) par simulation de la perception acoustique d'une personne dans cet espace en vue de déterminer l'existence d'une gêne acoustique pour cette personne, comprenant les étapes suivantes :

   - prendre avec au moins un capteur acoustique (1), notamment un capteur acoustique binaural, dans l'espace prédéfini (2) un échantillon acoustique sur une durée prédéfinie d'au moins une minute,
   - convertir l'échantillon acoustique en un flux de données correspondant à un flux de signaux du type neuronal arrivant au cerveau avec des canaux fréquentiels compris entre 16Hz et 16000Hz, le flux de signaux du type neuronal pour chaque canal fréquentiel étant formé par des impulsions dont la fréquence de répétition par seconde est une fonction de l'intensité du signal acoustique du canal fréquentiel considéré
   - évaluer pour chaque canal le flux de signaux du type neuronal selon une échelle de valeur biologique en déterminant un indice de nuisance acoustique pour chaque canal,
   - sélectionner un profil de caractérisation de ladite personne comprenant des caractéristiques physiologiques, temporelles et psychiques,
   - sélectionner un profil de caractérisation de l'espace comprenant des caractéristiques d'une activité présente dans l'espace et d'un lieu,
   - fournitures des indices de nuisance acoustique des canaux fréquentiels ainsi que du profil de caractérisation de ladite personne et du profil de caractérisation de l'espace en entrée d'un réseau neuronal artificiel pour évaluer de manière auditive l'espace prédéfini (2) par une classification selon au moins une première catégorie correspondant à la survenue d'une gêne acoustique perçue par la personne et une seconde catégorie correspondant à la perception d'une absence d'une gêne acoustique perçue par la personne.

2. Procédé selon la revendication 1, dans lequel on utilise 620 canaux fréquentiels compris entre 16Hz et 16000 Hz.

3. Procédé selon la revendication 1 ou 2, dans lequel la fréquence de répétition est comprise entre 0 et 330 par seconde.

4. Procédé selon l'une quelconque des revendications 1 à 3, dans lequel les caractéristiques physiologiques comprennent au moins un des paramètres suivants : l'âge, notamment une tranche d'âge, le sexe.

5. Procédé selon l'une quelconque des revendications 1 à 4, dans lequel les caractéristiques temporelles comprennent au moins un des paramètres suivants : mois de l'année, tranche horaire de la journée telle que matin, après-midi, soirée ou nuit.

6. Procédé selon l'une quelconque des revendications 1 à 5, dans lequel les caractéristiques psychiques comprennent au moins un des paramètres suivants : un état attentionnel tel que éveillé, somnolent ou endormi et un état émotionnel tel que calme/ serein, stressé /angoissé, joyeux / gai, triste / mélancolique ou irrité / en colère.

7. Procédé selon l'une quelconque des revendication 1 à 6, dans lequel l'échelle de valeur biologique est représentée par une fonction du type sigmoïde :

$$f(x) = \frac{1}{1+e^{(-\alpha(x-x_0)}}$$

- où x est l'intensité d'un stimulus, f(x) une réponse physiologique, $\alpha$ un facteur de sensibilité contrôlant la loi d'évolution et $x_o$ un point de basculement.

8. Procédé selon la revendication 7, dans lequel le facteur de sensibilité $\alpha$ est déterminé empiriquement par rapport à un protocole de test.

9. Procédé selon l'une quelconque des revendications 1 à 8, dans lequel les caractéristiques de l'activité comprennent au moins un des paramètres suivants : activité manuelle, sportive, ludique, musicale, informatique, lecture, écriture, discussion, méditation / réflexion.

10. Procédé selon l'une quelconque des revendications 1 à 9, dans lequel les caractéristiques de lieu comprennent au moins un des paramètres suivants : bureau, école, salle de réunion, atelier, moyen de transport, salle de spectacle, salle de restaurant, salle de sport, plein air.

11. Produit de programme informatique comprenant une ou plusieurs séquences d'instructions stockées accessibles à un processeur et qui, lorsqu'il est exécuté par le processeur, permet à ce dernier d'effectuer les étapes du procédé selon l'une quelconque des revendications 1 à 10.

12. Procédé de réduction d'une gêne acoustique perçue par une personne dans un espace prédéfini (2), dans lequel

- selon une première étape, on procède à l'évaluation auditive de l'espace prédéfini (2) selon l'une quelconque des revendications 1 à 10,
- si le résultat de la première étape est classé selon une catégorie correspondant à la survenue d'une gêne acoustique perçue par la personne, on procède à la modification dudit espace, notamment par la pose / disposition d'éléments d'absorption acoustique dans cet espace,
- on réitère les première et seconde étapes jusqu'à obtenir comme résultat de la première étape un classement selon une catégorie correspondant à l'absence d'une gêne acoustique.

13. Dispositif de mise en œuvre d'un procédé selon l'une quelconque des revendications 1 à 10, comprenant :

- au moins un capteur acoustique (1), notamment un capteur acoustique binaural, configuré pour pouvoir prendre dans un espace prédéfini (2) un échantillon acoustique sur une durée prédéfinie d'au moins une minute,
- un convertisseur (5) de l'échantillon acoustique en un flux de données correspondant à un flux de signaux du type neuronal arrivant au cerveau avec des canaux fréquentiels compris entre 16Hz et 16000Hz, le flux de signaux du type neuronal pour chaque canal fréquentiel étant formé par des impulsions dont la fréquence de répétition par seconde est une fonction de l'intensité du signal acoustique du canal fréquentiel considéré
- une unité (7) d'évaluation pour évaluer pour chaque canal le flux de signaux du type neuronal selon une échelle de valeur biologique en déterminant un indice de nuisance acoustique pour chaque canal,
- une première unité (9) de sélection configurée pour sélectionner un profil de caractérisation de ladite personne comprenant des caractéristiques physiologiques, temporelles et psychiques,
- une seconde unité (11) de sélection configurée pour sélectionner un profil de caractérisation de l'espace comprenant des caractéristiques d'une activité présente dans l'espace et d'un lieu,
- une unité de traitement à réseau neuronal artificiel (13) configurée pour recevoir en entrée des indices de nuisance acoustique des canaux fréquentiels ainsi que des profils de caractérisation de ladite personne et de caractérisation de l'espace en entrée, l'unité de traitement à réseau neuronal artificiel (13) étant configurée pour évaluer de manière auditive l'espace prédéfini (2) par une classification selon au moins une première catégorie correspondant à la survenue d'une gêne acoustique perçue par la personne et une seconde catégorie correspondant à la perception d'une absence d'une gêne acoustique perçue par la personne.

14. Dispositif selon la revendication 13, dans lequel les canaux fréquentiels compris entre 16Hz et 16000 Hz comprennent 620 canaux.

15. Dispositif selon la revendication 13 ou 14, dans lequel la fréquence de répétition du convertisseur de l'échantillon acoustique est compris entre entre 0 et 330 par seconde pour chaque canal.

16. Dispositif selon l'une quelconque des revendications 13 à 15, dans lequel les caractéristiques physiologiques comprennent au moins un des paramètres suivants : l'âge, notamment une tranche d'âge, le sexe.

17. Dispositif selon l'une quelconque des revendications 13 à 16, dans lequel les caractéristiques temporelles comprennent au moins un des paramètres suivants : mois de l'année, tranche horaire de la journée telle que matin, après-midi, soirée ou nuit.

18. Dispositif selon l'une quelconque des revendications 13 à 17, dans lequel les caractéristiques psychiques comprennent au moins un des paramètres suivants : un état attentionnel tel que éveillé, somnolent ou endormi et un état émotionnel tel que calme/ serein, stressé /angoissé, joyeux / gai, triste / mélancolique ou irrité / en colère.

19. Dispositif selon l'une quelconque des revendications 13 à 18, dans lequel l'échelle de valeur biologique de l'unité d'évaluation biologique est représentée par une fonction du type sigmoïde :

$$f(x) = \frac{1}{1+e^{(-\alpha(x-x_0))}}$$

- où x est l'intensité d'un stimulus, f(x) la réponse physiologique, $\alpha$ un facteur de sensibilité contrôlant la loi d'évolution et $x_0$ le point de basculement.

[Fig 1]

Figure 1

[Fig 2]

Figure 2

[Fig 3]

Figure 3

Europäisches
Patentamt

European
Patent Office

Office européen
des brevets

**RAPPORT DE RECHERCHE EUROPEENNE**

Numéro de la demande

**EP 25 20 4789**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | ZHANG JUNHUI ET AL: "Sound quality evaluation and prediction for the emitted noise of axial piston pumps", APPLIED ACOUSTICS, ELSEVIER PUBLISHING, GB, vol. 145, 29 septembre 2018 (2018-09-29), pages 27-40, XP085546371, ISSN: 0003-682X, DOI: 10.1016/J.APACOUST.2018.09.015 * page 27 - page 29 * * page 36 - page 39 * ----- | 1-19 | INV. A61B5/12 H04R29/00 |
| A | CN 114 386 328 A (HANGZHOU BOSS ELECTRIC APPLIANCE CO LTD) 22 avril 2022 (2022-04-22) * alinéas [0017], [0075] * ----- | 1-19 | |
| A | LOPEZ-BALLESTER JESUS ET AL: "Enabling Real-Time Computation of Psycho-Acoustic Parameters in Acoustic Sensors Using Convolutional Neural Networks", IEEE SENSORS JOURNAL, IEEE, USA, vol. 20, no. 19, 18 mai 2020 (2020-05-18), pages 11429-11438, XP011807405, ISSN: 1530-437X, DOI: 10.1109/JSEN.2020.2995779 [extrait le 2020-09-02] * page 11429 * * page 11432 * ----- | 1-19 | DOMAINES TECHNIQUES RECHERCHES (IPC) A61B H04S H04R |

Le présent rapport a été établi pour toutes les revendications

| Lieu de la recherche | Date d'achèvement de la recherche | Examinateur |
|---|---|---|
| La Haye | 21 janvier 2026 | Vanderperren, Yves |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                                                                      
& : membre de la même famille, document correspondant

EPO FORM 1503 03.82 (P04C02)

**EP 4 717 165 A1**

## ANNEXE AU RAPPORT DE RECHERCHE EUROPEENNE
## RELATIF A LA DEMANDE DE BREVET EUROPEEN NO.

EP 25 20 4789

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-01-2026

| Document brevet cité au rapport de recherche | Date de publication | Membre(s) de la famille de brevet(s) | Date de publication |
|---|---|---|---|
| CN 114386328 A | 22-04-2022 | AUCUN | |

Pour tout renseignement concernant cette annexe : voir Journal Officiel de l'Office européen des brevets, No.12/82

EPO FORM P0460

14